# EUROPEAN PATENT APPLICATION

(11) **EP 3 205 303 A1**
(43) Date of publication of application: **16.08.2017**
(21) Application number: 15872620.8
(22) Date of filing: 27.11.2015
(51) Int. Cl.: A61B 18/12

(54) **GRASPING TREATMENT UNIT AND GRASPING TREATMENT TOOL**

(30) Priority: 24.12.2014 JP 2014260464
(71) Applicant: Olympus Corporation, Hachioji-shi, Tokyo 192-8507 (JP)
(72) Inventor: TAKASHINO, Tomoyuki, Hachioji-shi, Tokyo 192-8507 (JP); TAKEI, Yusuke, Hachioji-shi, Tokyo 192-8507 (JP); TANAKA, Kazuhiro, Hachioji-shi, Tokyo 192-8507 (JP)
(74) Representative: Schicker, Silvia
(86) International application number: PCT/JP2015/083419
(87) International publication number: WO 2016/104051

(57) **Abstract**

In a grasping treatment unit in which a first jaw is openable and closable relative to a second jaw, a frame member is provided in the first jaw. The frame member includes a plate-shaped base portion forming a grasping surface side end face in the frame member, and a column extension extending from the base portion toward a back surface in the frame member and having one end continuous with the base portion. The column extension defines a space in which the frame member is a defining surface between the base portion and the back surface of the first jaw.

## Description

### Technical Field

The present invention relates to a grasping treatment unit to treat a grasped treated target, and a grasping treatment instrument including the grasping treatment unit.

### Background Art

Patent Literature 1 discloses a grasping treatment instrument which grasps a treated target between two jaws. This grasping treatment instrument treats the treated target grasped between one jaw and the other jaw by use of heat generated in a heat generator provided in one jaw. The treated target grasped between the two jaws is treated by use of heat. The jaw provided with the heat generator is formed into a continuous wall shape (solidly) without space in open and close directions from a grasping surface to a back surface.

### Citation List

### Patent Literature

Patent Literature 1: U.S. Patent No. 7329257

### Summary of Invention

### Technical Problem

In the above grasping treatment instrument according to Patent Literature 1, one jaw is continuous and solid without space in the open and close directions from the grasping surface to the back surface, so that heat generated in the heat generator is more easily transferred to the back surface as well as to the grasping surface where a treatment is conducted. When the treated target is treated by use of energy (e.g. a high-frequency) other than the heat energy as well, heat generated in the vicinity of the grasping surface in a treatment is more easily transferred to the back surface (parts other than the grasping surface in the outer surface of the jaw) in the configuration that is continuous without space in the open and close directions from the grasping surface to the back surface in the jaw. The heat for use in the treatment and the heat generated by the treatment are more easily transferred to the parts other than the grasping surface in the outer surface, so that living tissues and the like other than the treated target are subject to the heat.

For example, parts of the jaw other than the outer frame are formed into a cavity (i.e. the jaw is hollowed out) so that the volume of the jaw is decreased, whereby the transfer of the heat to the parts of the outer surface other than the grasping surface becomes more difficult. However, the rigidity of the jaw is reduced by the decrease of the volume of the jaw. Due to the reduction of the rigidity of the jaw, the jaw is deformed in a state where the treated target is grasped between the two jaws and the jaw is loaded. This leads to the reduction in the amount of force to grasp the treated target and the like, and the deterioration of treatment performance.

The present invention has been made in view of the above problems, and its object is to provide a grasping treatment unit and a grasping treatment instrument to maintain low performance of heat transfer to parts other than a grasping surface in the outer surface of a jaw and ensure the rigidity of the jaw.

### Solution to Problem

To solve above mentioned problems, according to one aspect of the invention, a grasping treatment unit includes that: a first jaw extending from a proximal portion toward a distal portion; a second jaw which extends from a proximal portion toward a distal portion, and which is openable and closable relative to the first jaw; a grasping surface facing the second jaw in an outer surface of the first jaw; a back surface facing a side opposite to the grasping surface in the outer surface of the first jaw; a frame member provided in the first jaw; a plate-shaped base portion which forms a grasping surface side end face in the frame member; and a column extension which extends from the base portion toward the back surface in the frame member, and which has one end continuous with the base portion, the column extension defining a space in which the frame member is a defining surface between the base portion and the back surface of the first jaw.

### Advantageous Effects of Invention

According to the present invention, a grasping treatment unit and a grasping treatment instrument, to maintain low performance of heat transfer to parts other than a grasping surface in the outer surface of a jaw and ensure the rigidity of the jaw, can be provided.

### Brief Description of Drawings

FIG. 1 is a schematic diagram showing a grasping treatment system according to a first embodiment;
FIG. 2 is a sectional view schematically showing the configuration of a distal portion of a grasping treatment instrument including a grasping treatment unit according to the first embodiment in a state where the space between a first jaw and a second jaw is open;
FIG. 3 is a sectional view schematically showing the grasping treatment unit according to the first embodiment in a section perpendicular to a longitudinal axis in a state where the space between the first jaw and the second jaw is closed;
FIG. 4 is a sectional view schematically showing the first jaw according to the first embodiment in a section perpendicular to width directions;
FIG. 5 is a sectional view schematically showing the first jaw according to the first embodiment in a section perpendicular to a first jaw axis;
FIG. 6 is a sectional view taken along the line VI-VI in FIG. 5;
FIG. 7 is a sectional view schematically showing the first jaw according to a first modification of the first embodiment in the section perpendicular to the first jaw axis;
FIG. 8 is a sectional view taken along the line VIII-VIII in FIG. 7;
FIG. 9 is a sectional view schematically showing the first jaw according to a second modification of the first embodiment in the section perpendicular to the first jaw axis;
FIG. 10 is a sectional view taken along the line X-X in FIG. 9;
FIG. 11 is a sectional view schematically showing the first jaw according to a third modification of the first embodiment in the section perpendicular to the first jaw axis;
FIG. 12A is a sectional view schematically showing the first jaw according to a fourth modification of the first embodiment in the section perpendicular to open and close directions of the first jaw;
FIG. 12B is a schematic diagram showing the configuration of a base portion of a frame member of the first jaw according to a fifth modification of the first embodiment;
FIG. 12C is a schematic diagram showing the configuration of the base portion of the frame member of the first jaw according to a sixth modification of the first embodiment;
FIG. 13 is a sectional view schematically showing the first jaw according to a second embodiment in the section perpendicular to the width directions;
FIG. 14 is a sectional view schematically showing the first jaw according to the second embodiment in the section perpendicular to the first jaw axis;
FIG. 15 is a sectional view taken along the line XV-XV in FIG. 14;
FIG. 16 is a sectional view schematically showing the grasping treatment unit according to a first modification of the first embodiment and the second embodiment in the section perpendicular to the longitudinal axis;
FIG. 17 is a sectional view schematically showing the grasping treatment unit according to a second modification of the first embodiment and the second embodiment in the section perpendicular to the longitudinal axis;
FIG. 18 is a sectional view schematically showing the grasping treatment unit according to a third modification of the first embodiment and the second embodiment in the section perpendicular to the longitudinal axis;
FIG. 19 is a sectional view schematically showing the first jaw according to a third embodiment in the section perpendicular to the width directions;
FIG. 20 is a sectional view schematically showing the first jaw according to a fourth embodiment in the section perpendicular to the width directions; and
FIG. 21 is a sectional view schematically showing the first jaw according to a fifth embodiment in the section perpendicular to the width directions.

### Description of Embodiments

### (First Embodiment)

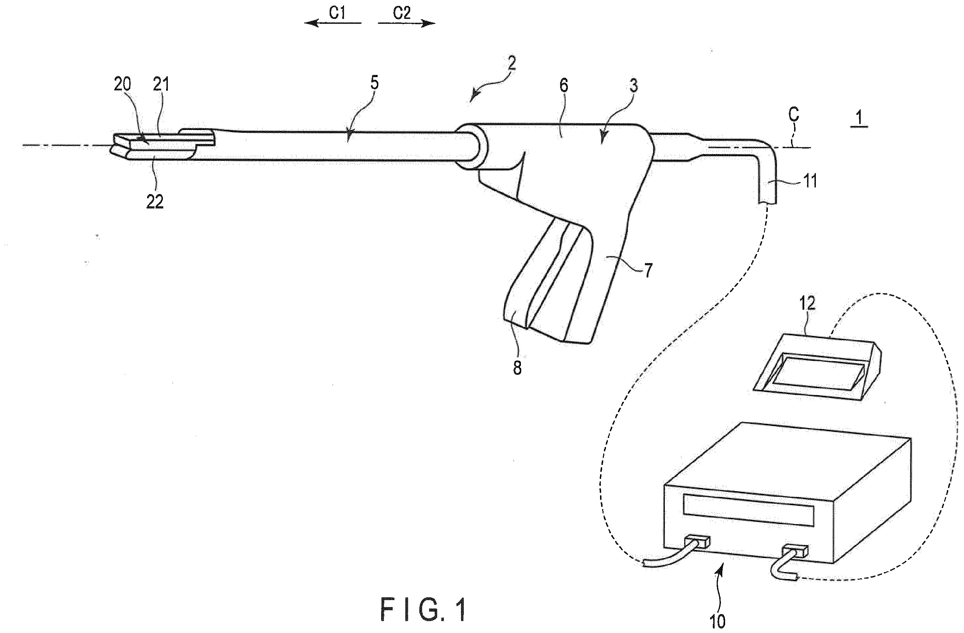

A first embodiment of the present invention will be described with reference to FIG. 1 to FIG. 6.

FIG. 1 is a diagram showing a grasping treatment system 1. As shown in FIG. 1, the grasping treatment system 1 includes a grasping treatment instrument 2. The grasping treatment instrument 2 has a longitudinal axis C. Here, a direction parallel to the longitudinal axis C is a longitudinal axis direction. One side in the longitudinal axis direction is a distal side (an arrow C1 side in FIG. 1), and the side opposite to the distal side is a proximal side (an arrow C2 side in FIG. 1). In the present embodiment, the grasping treatment instrument 2 is a heat treatment instrument configured to treat a treated target such as a living tissue by using heat as energy, and is also a high-frequency treatment instrument configured to treat the treated target by using high-frequency electric power (high-frequency current).

The grasping treatment instrument 2 includes a holding unit (handle unit) 3 which can be held by a surgeon, and a cylindrical shaft (sheath) 5 which is coupled to the distal side of the holding unit 3. In the present embodiment, the central axis of the shaft 5 is the longitudinal axis C. The holding unit 3 includes a case body 6 extending along the longitudinal axis C, and a fixed handle 7 extending from the case body 6 toward a certain direction that intersects with the longitudinal axis C. In the present embodiment, the case body 6 is provided coaxially with the shaft 5, and the shaft 5 is inserted into the case body 6 from the distal side and thereby attached to the holding unit 3. The fixed handle 7 is formed integrally with the case body 6. The holding unit 3 includes a movable handle 8 which is rotatably attached to the case body 6. The movable handle 8 is rotated relative to the case body 6, and the movable handle 8 is thereby moved to open or close relative to the fixed handle 7.

One end of a cable 11 is connected to the holding unit 3 (the case body 6). The grasping treatment system 1 includes an energy source unit 10 which is, for example, an energy control device. The other end of the cable 11 is connected to the energy source unit 10. The energy source unit 10 includes an electric power supply, a conversion circuit which converts electric power from the electric power supply into high-frequency electric power, and a conversion circuit which converts electric power from the electric power supply into heat generating electric power. The energy source unit includes a processor which includes a central processing unit (CPU) or an application specific integrated circuit (ASIC), and a storage such as a memory. The energy source unit 10 is electrically connected to an energy operation input portion 12 such as a foot switch.

A grasping treatment unit (end effector) 20 is coupled to the distal side of the shaft 5. The grasping treatment unit 20 includes a first jaw 21 which is a first grasping portion, and a second jaw 22 which is a second grasping portion. In the grasping treatment unit 20, the space between the first jaw 21 and the second jaw 22 is openable and closable. That is, the first jaw 21 and the second jaw 22 are openable and closable relative to each other.

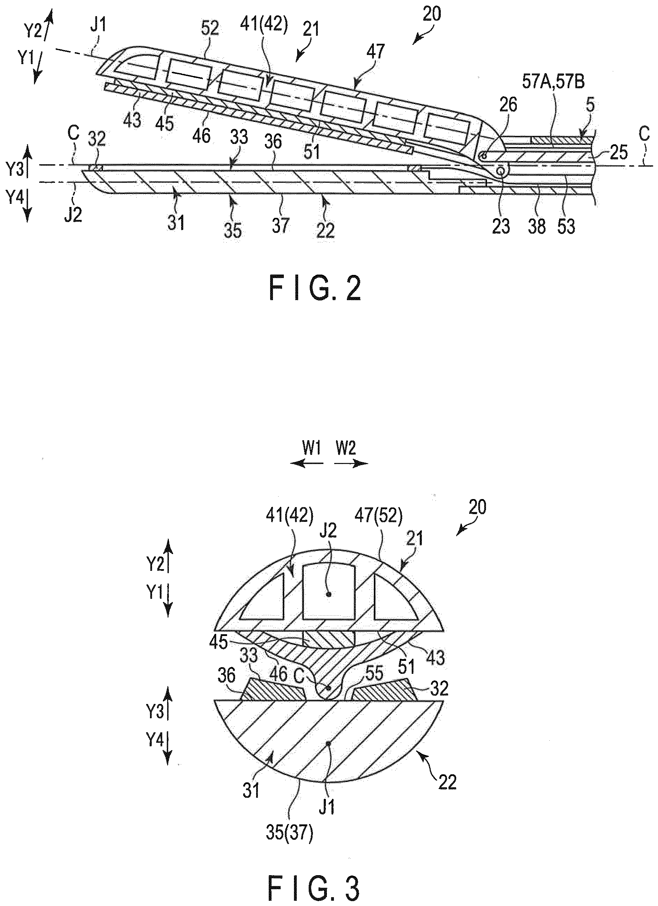

FIG. 2 is a diagram showing the configuration of the distal portion of the grasping treatment instrument 2 including the grasping treatment unit 20. FIG. 2 shows a state where the space between the first jaw 21 and the second jaw 22 is open. FIG. 3 shows the first jaw 21 and the second jaw 22 in a section perpendicular to the longitudinal axis C. In FIG. 3, the space between the first jaw 21 and the second jaw 22 is closed.

As shown in FIG. 2 and FIG. 3, the first jaw 21 has a first jaw axis J1. The first jaw axis J1 is the central axis of the first jaw 21, and the first jaw (first grasping portion) 21 extends along the first jaw axis J1 from the proximal portion toward the distal portion. Here, a direction parallel to the first jaw axis J1 is a longitudinal direction of the first jaw 21 (first jaw longitudinal direction). One side in the longitudinal direction is a distal side of the first jaw 21 (first jaw distal direction), and the side opposite to the distal side (first jaw distal direction) is a proximal side of the first jaw 21 (first jaw proximal direction). The distal side of the first jaw 21 corresponds to a side toward the distal portion in the first jaw 21, and the proximal side of the first jaw 21 corresponds to a side toward the proximal portion in the first jaw 21.

The second jaw 22 has a second jaw axis J2. The second jaw axis J2 is the central axis of the second jaw 22, and the second jaw (second grasping portion) 22 extends along the second jaw axis J2 from the proximal portion toward the distal portion. Here, a direction parallel to the second jaw axis J2 is a longitudinal direction of the second jaw 22 (second jaw longitudinal direction). One side in the longitudinal direction is a distal side of the second jaw 22 (second jaw distal direction), and the side opposite to the distal side (second jaw distal direction) is a proximal side of the second jaw 22 (second jaw proximal direction). The distal side of the second jaw 22 corresponds to a side toward the distal portion in the second jaw 22, and the proximal side of the second jaw 22 corresponds to a side toward the proximal portion in the second jaw 22.

In the present embodiment, the second jaw 22 is fixed to the shaft 5 in the distal portion of the shaft 5. The second jaw axis J2 is substantially parallel to the longitudinal axis C of the shaft 5. The first jaw 21 is attached to the distal portion of the shaft 5 via a support pin 23. The first jaw 21 is rotatable relative to the shaft 5 around the support pin 23. A rod 25 extends from the proximal side to the distal side inside the shaft 5. The rod 25 is movable relative to the shaft 5 along the longitudinal axis C. The proximal portion of the rod 25 is coupled to the movable handle 8 inside the case body 6. The distal portion of the rod 25 is connected to the first jaw 21 via a connection pin 26. The movable handle 8 is moved to open or close relative to the fixed handle 7, and the rod 25 is thereby moved relative to the shaft 5 along the longitudinal axis C. As a result, the first jaw 21 rotates relative to the shaft 5, and the first jaw 21 is moved to open or close relative to the second jaw 22. In this instance, the second jaw 22 opens or closes relative to the first jaw 21 because the second jaw 22 is fixed to the shaft 5. That is, the space between the first jaw 21 and the second jaw 22 opens or closes in the grasping treatment unit 20 in response to the movement of the rod 25 relative to the shaft 5. Therefore, the movable handle 8 is an open-or-close operation input portion configured to input an open-or-close operation to open or close the space between the first jaw (first grasping portion) 21 and the second jaw (second grasping portion) 22.

Here, a direction toward the second jaw 22 in the first jaw 21 is a close direction (direction of an arrow Y1 in FIG. 2 and FIG. 3) of the first jaw 21, and the direction away from the second jaw 22 in the first jaw 21 is an open direction (direction of an arrow Y2 in FIG. 2 and FIG. 3) of the first jaw 21. The close direction (first jaw close direction) of the first jaw 21 is one direction that intersects with (perpendicular to) the first jaw axis J1, and the open direction (first jaw open direction) of the first jaw 21 is a direction opposite to the jaw close direction. A direction toward the first jaw 21 in the second jaw 22 is a close direction (direction of an arrow Y3 in FIG. 2 and FIG. 3) of the second jaw 22, and the direction away from the first jaw 21 in the second jaw 22 is an open direction (direction of an arrow Y4 in FIG. 2 and FIG. 3) of the second jaw 22. The close direction (second jaw close direction) of the second jaw 22 is one direction intersecting with (perpendicular to) the second jaw axis J2, and the open direction (second jaw open direction) of the second jaw 22 is a direction opposite to the jaw close direction. Directions intersecting with (perpendicular to) the first jaw axis J1 and perpendicular to the open direction and close direction of the first jaw 21 are width directions (directions of an arrow W1 and an arrow W2 in FIG. 3). The width directions (jaw width directions) are directions intersecting with (perpendicular to) the second jaw axis J2 and perpendicular to the open direction and close direction of the second jaw 22.

The second jaw 22 includes a jaw main body (second jaw main body) 31 which is a support member fixed to the shaft 5, and an electrode portion (second electrode portion) 32 fixed to the jaw main body 31. The jaw main body 31 and the electrode portion 32 extend along the second jaw axis J2 from the distal portion to the proximal portion of the second jaw 22. The outer surface of the jaw main body 31 is made of an electrically insulating material, and the electrode portion 32 is made of an electrically conductive material. The outer surface of the second jaw 22 is provided with a grasping surface (second jaw grasping surface) 33 facing the first jaw 21, and a back surface (second jaw back surface) 35 facing in the direction opposite to the grasping surface 33 (i.e. in the open direction of the second jaw 22). The jaw main body (support member) 31 has a grasping surface side end face (close direction side end face) 36 and a back surface side end face (open direction side end face) 37. In the present embodiment, the jaw main body 31 is formed into a continuous wall shape (solidly) without space from the grasping surface side end face 36 to the back surface side end face 37. In the present embodiment, the back surface 35 of the second jaw 22 is formed by the back surface side end face (second back surface side end face) 37 of the jaw main body 31, and the grasping surface 33 of the second jaw 22 is formed by part of the grasping surface side end face (second grasping surface side end face) 36 of the jaw main body 31 and the electrode portion 32.

One end of an electric power supply line (second high-frequency electric power supply line) 38 formed by, for example, an electric wire is connected to the electrode portion 32. The electric power supply line 38 extends through the space between the shaft 5 and the rod 25, an inside of the case body 6 and an inside of the cable 11, and has the other end connected to the energy source unit 10. The energy source unit 10 can output high-frequency electric power (high-frequency electric energy), and the high-frequency electric power output from the energy source unit 10 is supplied to the electrode portion 32 of the second jaw 22 through the electric power supply line 38. When the electric power is supplied to the electrode portion 32, the electrode portion 32 functions as one electrode (second electrode) of the high-frequency electric power. Because the outer surface of the jaw main body (support member) 31 is made of the electrically insulating material, no high-frequency electric power is supplied (transmitted) to the jaw main body 31.

As shown in FIG. 2 and FIG. 3, the first jaw 21 includes a jaw main body (first jaw main body) 41 attached to the shaft 5 and the rod 25. In the present embodiment, the jaw main body 41 is only formed by a frame member 42. The frame member (support member) 42 extends along the first jaw axis J1 from the proximal portion to the distal portion of the first jaw 21. The frame member 42 is made of a metal including at least one of stainless and titanium, or a heat-resistant resin including at least one of polyether ether ketone (PEEK) and polyimide (PI), and the outer surface of the frame member 42 is made of the electrically insulating material. The first jaw 21 includes a blade (energy feeding portion) 43, and a heat generator (heating element) 45 which generates heat. The blade 43 and the heat generator 45 extend along the first jaw axis J1 from the proximal portion to the distal portion of the first jaw 21. The blade 43 is made of a material (e.g. copper) which is electrically conductive and which is high in thermal conductivity. The outer surface of the first jaw 21 is provided with a grasping surface (first jaw grasping surface) 46 facing the second jaw 22, and a back surface (first jaw back surface) 47 facing the side opposite to the grasping surface 46 (i.e. in the open direction of the first jaw 21). The blade 43 is coupled (fixed) to the grasping surface side (close direction side) of the frame member 42 (jaw main body 41), and the heat generator 45 is provided between the frame member 42 and the blade 43 in the open and close directions of the first jaw 21. The frame member (support member) 42 has a grasping surface side end face (close direction side end face) 51 and a back surface side end face (open direction side end face) 52. In the present embodiment, the back surface 47 of the first jaw 21 is formed by the back surface side end face (first back surface side end face) 52 of the frame member 42 (jaw main body 41). The heat generator 45 is disposed between the grasping surface side end face (second grasping surface side end face) 51 of the frame member 42 and the blade 43. The grasping surface 46 of the first jaw 21 is formed by the blade 43.

One end of an electric power supply line (first high-frequency electric power supply line) 53 formed by, for example, an electric wire is connected to the proximal portion of the blade (energy feeding portion) 43. The electric power supply line 53 extends through the space between the shaft 5 and the rod 25, the inside of the case body 6 and the inside of the cable 11, and has the other end connected to the energy source unit 10. High-frequency electric power output from the energy source unit 10 is supplied to the blade 43 of the first jaw 21 through the electric power supply line 53. When the electric power is supplied to the blade 43, the blade 43 functions as an electrode (first electrode) of the high-frequency electric power different in electric potential from the electrode portion 32. The blade 43 feeds the high-frequency electric power to the treated target from the grasping surface 46 as energy for use in a treatment. Because the outer surface of the frame member 42 is made of the electrically insulating material, no high-frequency electric power is supplied (transmitted) to the frame member 42.

The blade 43 can abut on the grasping surface side end face (second grasping surface side end face) 36 of the jaw main body (second jaw main body) 31 in a state where the space between the first jaw 21 and the second jaw 22 is closed. Therefore, the part of the grasping surface side end face 36 that forms the grasping surface 33 of the second jaw 22 is an abutment receiving portion 55 on which the blade 43 can abut. When the space between the first jaw 21 and the second jaw 22 is closed in a state no treated target is present between the first jaw 21 and the second jaw 22, the blade 43 abuts on the abutment receiving portion 55. In a state where the blade 43 is in abutment with the abutment receiving portion 55, the blade 43 does not contact the electrode portion 32 of the second jaw 22, and there is a gap between the blade 43 and the electrode portion 32. This prevents the contact between the electrode portion 32 of the second jaw 22 and the blade 43 of the first jaw 21 that are different in electric potential from each other.

One end of each of an electric power supply line (first thermoelectric power supply line) 57A and an electric power supply line (second thermoelectric power supply line) 57B that are formed by, for example, electric wires is connected to the heat generator 45. The electric power supply lines 57A and 57B extend through the space between the shaft 5 and the rod 25, the inside of the case body 6 and the inside of the cable 11, and have the other ends connected to the energy source unit 10. The energy source unit 10 can output heat generating electric power (heat generating electric energy) to be supplied to the heat generator 45, in addition to the aforementioned high-frequency electric power. The heat generating electric power from the energy source unit 10 is supplied to the heat generator 45 of the first jaw 21 through the electric power supply lines 57A and 57B. Heat is generated by the supply of the electric power to the heat generator 45. The heat generated in the heat generator 45 is transferred to the grasping surface (first grasping surface) 46 through the blade 43, and the heat is fed to the treated target from the grasping surface 46 as energy for use in a treatment.

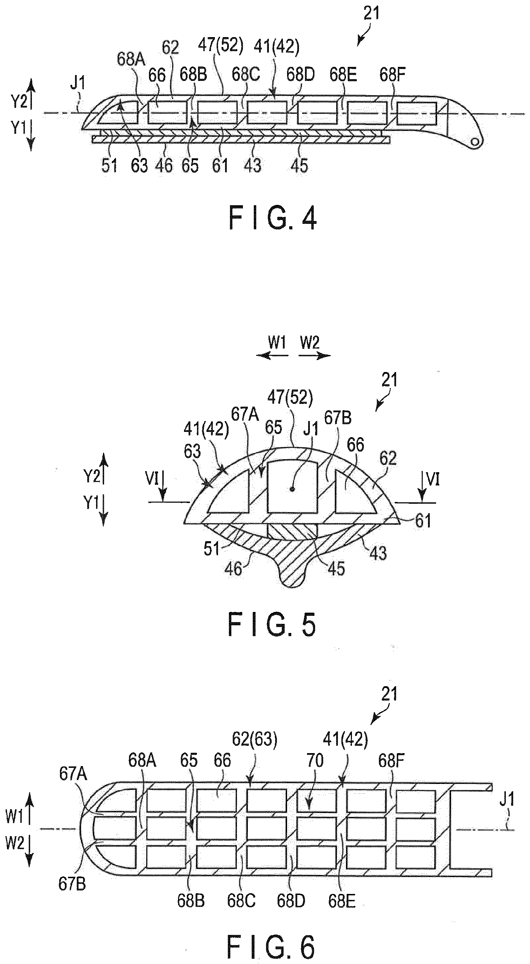

FIG. 4 to FIG. 6 are diagrams showing the configuration of the first jaw 21. FIG. 4 shows a section of the first jaw 21 perpendicular to the width directions. FIG. 5 shows a section of the first jaw 21 perpendicular to the first jaw axis J1. FIG. 6 is a sectional view taken along the line VI-VI in FIG. 5, and shows a section of the first jaw 21 perpendicular to the open and close directions. As shown in FIG. 4 to FIG. 6, the frame member 42 includes a plate-shaped base portion 61. The grasping surface side end face (close direction side end face) 51 of the frame member 42 (the jaw main body 41) is formed by the base portion 61. The frame member 42 also includes a frame portion 62 having both ends continuous with the base portion 61. In the present embodiment, the frame portion 62 is substantially semicircular-arc-shaped in a section perpendicular to the first jaw axis J1. An outer frame 63 of the frame member 42 is formed by the base portion 61 and the frame portion 62. The frame portion 62 forms the back surface side end face (open direction side end face) 52 of the frame member 42. In the present embodiment, the back surface 47 of the first jaw 21 is formed by the frame portion 62 because the back surface 47 of the first jaw 21 is formed by the back surface side end face 52 of the frame member 42. The base portion 61 and the frame portion 62 are parts of the frame member 42, and are made of a metal including at least one of stainless and titanium, or a heat-resistant resin including at least one of polyether ether ketone (PEEK) and polyimide (PI).

A column extension 65 extends inside the outer frame 63 in the open and close directions of the first jaw 21. Inside the outer frame 63 (the frame member 42), a space 66 is formed in a part where the column extension 65 does not extend. Therefore, inside the outer frame 63, the space 66 in which the frame member 42 (the outer frame 63 and the column extension 65) is a defining surface is defined. That is, the space 66 in which the frame member 42 is the defining surface is defined by the column extension 65 between the base portion 61 and the back surface 47 of the first jaw 21. One end (grasping surface side end) of the column extension 65 is continuous with the base portion 61. In the present embodiment, the other end (back surface side end) of the column extension 65 is continuous with the frame portion 62. Thus, in the present embodiment, both ends of the column extension 65 are continuous with the outer frame 63.

The column extension 65 is formed by axial direction elements 67A and 67B extending along the first jaw axis J1, and width direction elements 68A to 68F extending along the width directions of the first jaw 21 (directions of an arrow W1 and an arrow W2 in each of FIGS. 5 and 6). Therefore, in the present embodiment, a lattice structure 70 is formed by the column extension 65 in the section perpendicular to the open and close directions of the first jaw 21 (directions of an arrow Y1 and an arrow Y2 in each of FIGS. 5 and 6).

Next, functions and advantageous effects of the grasping treatment unit 20 and the grasping treatment instrument 2 according to the present embodiment are described. When a treated target such as a living tissue is treated by use of the grasping treatment system 1, the grasping treatment unit 20 (the first jaw 21 and the second jaw 22) is inserted into the body, and the treated target is disposed between the first jaw 21 and the second jaw 22. The movable handle 8 is then moved to close relative to the fixed handle 7, and a close operation of the grasping treatment unit 20 is input. As a result, the space between the first jaw 21 and the second jaw 22 is closed, and the treated target is grasped between the first jaw 21 and the second jaw 22. In a state where the treated target is grasped, an energy operation is input in the energy operation input portion 12. Accordingly, heat generating electric power is output from the energy source unit 10, and high-frequency electric power is output.

When the heat generating electric power is supplied to the heat generator 45 from the energy source unit 10, heat is generated in the heat generator 45, and the generated heat is transferred to the grasping surface (first grasping surface) 46 formed in the blade 43 of the first jaw 21. As a result, the treated target which is in abutment with the grasping surface 46 is burned, and the treated target is cut open. When high-frequency electric power is supplied to the electrode portion 32 of the second jaw 22 and the blade 43 of the first jaw 21 from the energy source unit 10, the electrode portion 32 and the blade 43 function as electrodes that are different in electric potential from each other. As a result, a high-frequency electric current flows between the electrode portion 32 and the blade 43 through the treated target grasped between the first jaw 21 and the second jaw 22. The high-frequency electric current denatures the treated target, and accelerates coagulation.

In this instance, the heat generated in the heat generator 45 is transferred to the grasping surface 46 side, and also transferred to the back surface 47 side. Heat is generated in the vicinity of the grasping surface 46 by the feed of high-frequency electric power to the treated target from the grasping surface 46. Thus, the heat resulting from the high-frequency electric power is transferred to the back surface 47 side. In the present embodiment, in the frame member 42 of the jaw main body 41, the space 66 in which the frame member 42 is the defining surface is defined inside the outer frame 63. That is, the frame member 42 (the jaw main body 41) is not continuous without space from the grasping surface side end face 51 to the back surface side end face 52 in the open and close directions of the first jaw 21, and the space 66 is formed as an air layer between the grasping surface side end face 51 and the back surface side end face 52. Thus, low performance of heat transfer is maintained inside the frame member 42, and it is difficult for the heat generated in the heat generator 45 (the heat for use in a treatment) and the heat generated in the vicinity of the grasping surface 46 by high-frequency electric power (the heat generated by the treatment) to be transferred to the back surface 47 side through the frame member 42. Therefore, it is difficult for heat to be transferred to parts other than the grasping surface 46 in the outer surface of the first jaw 21 in a state where a treatment is conducted in the vicinity of the grasping surface 46. That is, low performance of heat transfer to parts other than the grasping surface 46 is maintained in the outer surface of the first jaw 21. This can reduce the influence of the heat for use in the treatment and the heat generated by the treatment to a living tissue or the like other than the treated target.

Moreover, in the present embodiment, the column extension 65 extends inside the outer frame 63 in the frame member 42, and one end (grasping surface side end) of the column extension 65 is continuous with the base portion 61. Thus, even if the space 66 is formed in the outer frame 63, the rigidity of the frame member 42 is not lower, and the rigidity of the first jaw 21 is not lower either. Because high rigidity of the first jaw 21 is maintained, the deformation of the first jaw 21 is difficult even if the treated target is grasped between the first jaw 21 and the second jaw 22 and the first jaw 21 is loaded. Consequently, a great grasping force amount is maintained in a state where the treated target is grasped between the first jaw 21 and the second jaw 22, and treatment performance can be ensured.

In the present embodiment, the outer frame 63 of the frame member 42 is formed by the frame portion 62 and the base portion 61, and the other end (back surface side end) of the column extension 65 is continuous with the frame portion 62. Thus, the rigidity of the frame member 42 (the jaw main body 41) is higher, and the rigidity of the first jaw 21 is higher. In the present embodiment, a lattice structure 68 is formed by the column extension 65 in the section perpendicular to the open and close directions of the first jaw 21. Thus, the rigidity of the frame member 42 (the jaw main body 41) is higher, and the rigidity of the first jaw 21 is higher.

As described above, in the grasping treatment unit 20 according to the present embodiment, low performance of heat transfer to parts other than the grasping surface 46 is maintained in the outer surface of the first jaw 21, and the rigidity of the first jaw 21 is also ensured. In the grasping treatment instrument 2 having the first jaw 21 which is inserted into the body, the first jaw 21 needs to be reduced in size. In one example, the first jaw 21 needs to be formed so that its section perpendicular to the first jaw axis J1 may be within a diameter of 5 mm. Actually, when the shape of the section of the outer frame 63 perpendicular to the first jaw axis J1 is formed into a rectangular cylindrical shape, the first jaw 21 increases in size, so that as in the present embodiment, the shape of the section of the outer frame 63 perpendicular to the first jaw axis J1 is formed into a semicircular cylindrical shape, and the first jaw 21 is reduced in size. However, when the shape of the section of the outer frame 63 perpendicular to the first jaw axis J1 is formed into a semicircular cylindrical shape, strength against the load applied in grasping (force in the open and close directions of the first jaw 21) is lower in a monocoque construction in which the column extension 65 is not provided and in which the frame member 42 is only formed by the outer frame 63. Therefore, in the present embodiment, the column extension 65 is provided so that strength against the load applied in grasping is ensured even when the shape of the section of the outer frame 63 perpendicular to the first jaw axis J1 is formed into a semicircular cylindrical shape.

### (Modifications of First Embodiment)

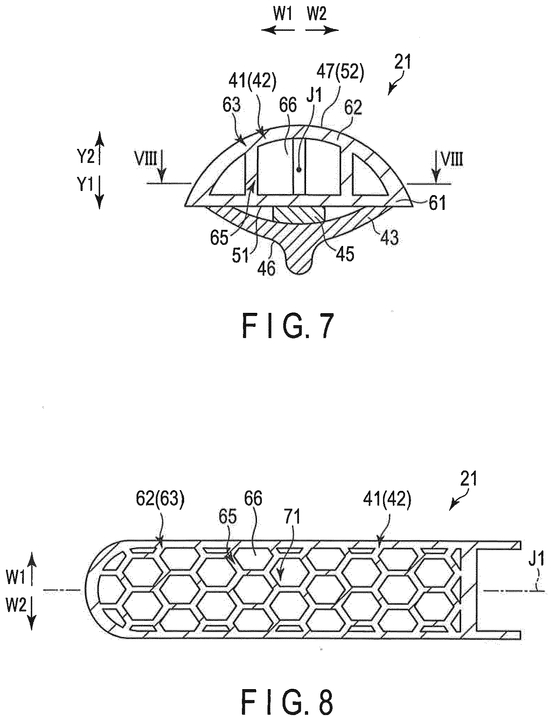

Although the lattice structure 70 is formed by the column extension 65 in the section perpendicular to the open and close directions of the first jaw 21 in the first embodiment, it is not limited to this. For example, in a first modification of the first embodiment shown in FIG. 7 and FIG. 8, a honeycomb structure 71 may be formed by the column extension 65 in the section perpendicular to the open and close directions of the first jaw 21. FIG. 7 shows the section of the first jaw 21 perpendicular to the first jaw axis J1. FIG. 8 shows a section taken along the line VIII-VIII in FIG. 7. In the present modification as well, the space 66 is formed in the part where the column extension 65 does not extend inside the outer frame 63 of the frame member 42. That is, the space 66 in which the frame member 42 is the defining surface is defined inside the outer frame 63. The column extension 65 has one end (grasping surface side end) continuous with the base portion 61, and the other end (back surface side end) continuous with the frame portion 62. With the configuration described above, in the present modification as well, low performance of heat transfer to parts other than the grasping surface 46 is maintained in the outer surface of the first jaw 21, and the rigidity of the first jaw 21 is also ensured.

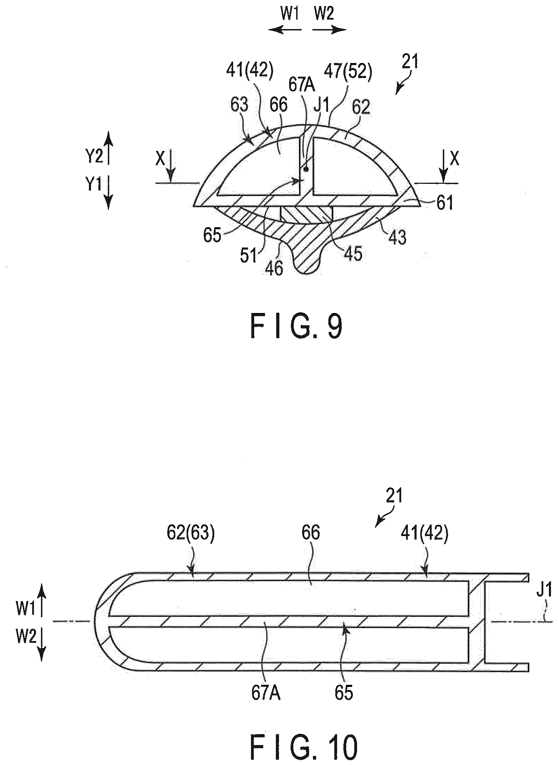

For example, in a second modification of the first embodiment shown in FIG. 9 and FIG. 10, the column extension 65 may be only formed by the axial direction element 67A extending along the first jaw axis J1. FIG. 9 shows the section of the first jaw 21 perpendicular to the first jaw axis J1. FIG. 10 shows a section taken along the line X-X in FIG. 9. In the present modification as well, the space 66 is formed in the part where the column extension 65 does not extend inside the outer frame 63 of the frame member 42. That is, the space 66 in which the frame member 42 is the defining surface is defined inside the outer frame 63. The column extension 65 has one end (grasping surface side end) continuous with the base portion 61, and the other end (back surface side end) continuous with the frame portion 62. With the configuration described above, in the present modification as well, low performance of heat transfer to parts other than the grasping surface 46 is maintained in the outer surface of the first jaw 21, and the rigidity of the first jaw 21 is also ensured.

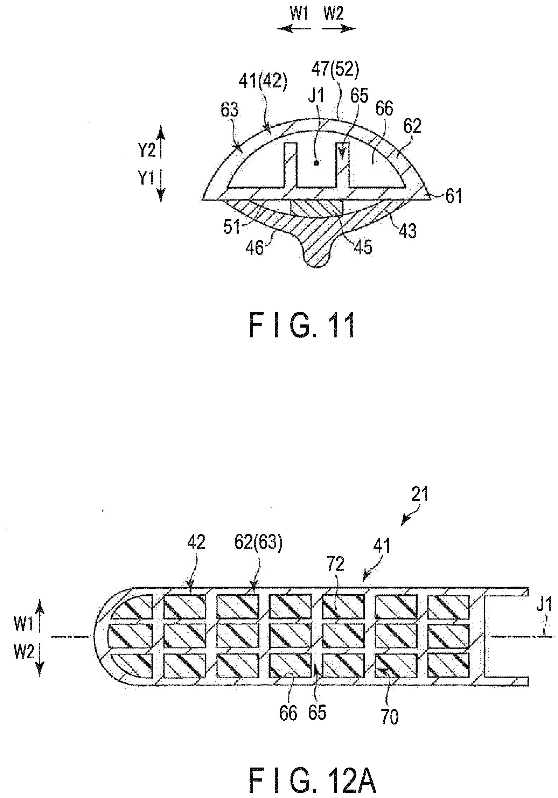

For example, in a third modification of the first embodiment shown in FIG. 11, the column extension 65 may only have one end (grasping surface side end) continuous with the base portion 61, and may have the other end (back surface side end) non-continuous with the frame portion 62. FIG. 11 shows a section of the first jaw 21 perpendicular to the first jaw axis J1. In the present modification, the space 66 is formed between the other end of the column extension 65 and the frame portion 62. Preferably, the column extension 65 extends along the first jaw axis J1 in the same manner as the axial direction element 67A shown in FIG. 9 and FIG. 10. In the present modification as well, one end (grasping surface side end) of the column extension 65 is continuous with the base portion 61, so that the rigidity of the frame member 42 is ensured. Therefore, in the present modification as well, low performance of heat transfer to parts other than the grasping surface 46 is maintained in the outer surface of the first jaw 21, and the rigidity of the first jaw 21 is also ensured.

For example, in a fourth modification of the first embodiment shown in FIG. 12A, the space 66 formed by the column extension 65 (i.e. the part where the column extension 65 does not extend) is filled with a filling material 72 inside the outer frame 63 of the frame member 42. In the present modification, the jaw main body (first jaw main body) 41 of the first jaw 21 is formed by the frame member 42 and the filling material 72. The filling material 72 is made of a heat-resistant material lower in thermal conductivity than the frame member 42. For example, the filling material 72 is made of a porous metal including at least one of steel, aluminum, stainless, and titanium, or a foamed resin including at least one of polyether ether ketone (PEEK) and polyimide (PI). In the present modification as well, one end (grasping surface side end) of the column extension 65 is continuous with the base portion 61.

In the present modification, the space 66 is formed inside the outer frame 63 in the frame member 42, and the space 66 is filled with the filling material 72. That is, the frame member 42 is not continuous without space from the grasping surface side end face 51 to the back surface side end face 52 in the open and close directions of the first jaw 21, and the space 66 in which the frame member 42 is the defining surface is defined between the grasping surface side end face 51 and the back surface side end face 52. The space 66 formed by the frame member 42 is filled with the filling material 72. The filling material 72 that fills the space 66 is low in thermal conductivity. Thus, in the present embodiment as well, it is difficult for heat to be transferred to parts other than the grasping surface 46 in the outer surface of the first jaw 21 in a state where a treatment is conducted in the vicinity of the grasping surface 46. Therefore, in the present modification as well, low performance of heat transfer to parts other than the grasping surface 46 is maintained in the outer surface of the first jaw 21, and the rigidity of the first jaw 21 is also ensured.

Although the lattice structure 70 is formed by the column extension 65 in the section perpendicular to the open and close directions of the first jaw 21 in the present modification, it is not limited to this. In the configuration in which the space 66 is filled with the filling material 72 as well, the honeycomb structure 71 may be formed by the column extension 65 in the section perpendicular to the open and close directions of the first jaw 21 as in the first modification of the first embodiment, or the column extension 65 may be only formed by the axial direction element 67A as in the second modification of the first embodiment. Moreover, in the configuration in which the space 66 is filled with the filling material 72 as well, the other end (back surface side end) of the column extension 65 may be non-continuous with the frame portion 62, as in the third modification of the first embodiment.

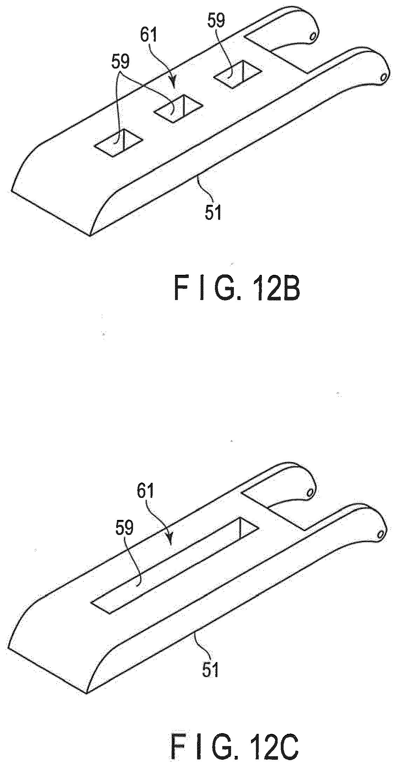

As in a fifth modification of the first embodiment shown in FIG. 12B and a sixth modification of the first embodiment shown in FIG. 12C, a hole 69 may be provided in the base portion 61 of the frame member 42. The hole 69 penetrates the base portion 61 in the open and close directions of the first jaw 21. As in the fifth modification, more than one hole may be formed in the base portion 61. As in the sixth modification, only one long hole 69 along the first jaw axis J1 may be formed in the base portion 61. When the hole 69 is provided, it is difficult for the heat from the heat generator 45 and the heat generated by the treatment to be transferred to the base portion 61 via the grasping surface side end face 51. This makes it possible to maintain lower performance of heat transfer to parts other than the grasping surface 46 in the outer surface of the first jaw 21.

In the first embodiment and its modifications, in the first jaw (21), the frame member (42) includes the plate-shaped base portion (61) which forms the grasping surface side end face (51) in the frame member (42), and the frame portion (62) having both ends continuous with the base portion (61). The base portion (61) and the frame portion (62) cooperate to form the outer frame (63) of the frame member (42), and the space (66) in which the frame member (42) is the defining surface is formed inside the outer frame (63). The column extension (65) extends inside the frame portion (62), and has one end continuous with the base portion (61).

### (Second Embodiment)

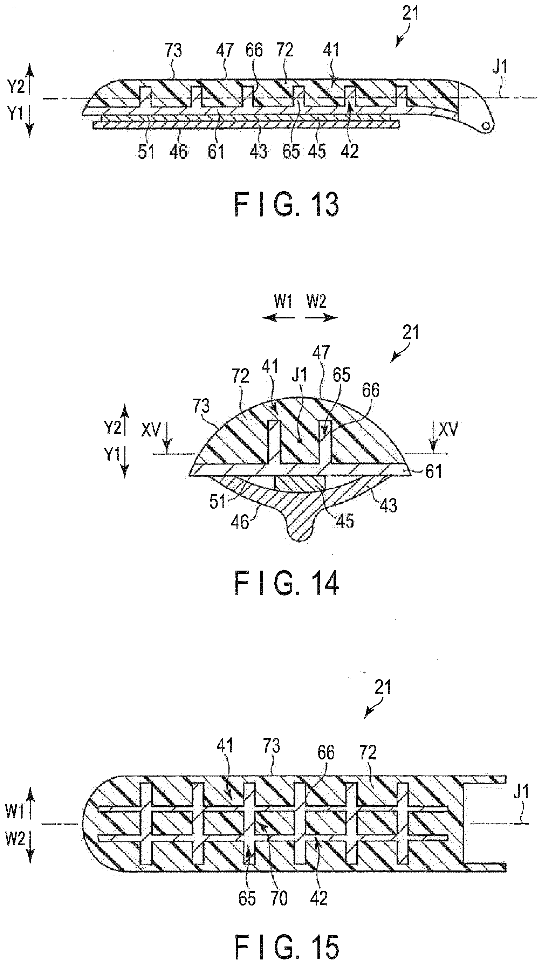

Next, a second embodiment of the present invention will be described with reference to FIG. 13 to FIG. 15. The second embodiment is the following modification of the configuration of the first embodiment. The same parts as those in the first embodiment are provided with the same reference marks, and are not described.

FIG. 13 to FIG. 15 are diagrams showing the configuration of the first jaw 21. FIG. 13 shows the section perpendicular to the width directions of the first jaw 21. FIG. 14 shows the section of the first jaw 21 perpendicular to the first jaw axis J1. FIG. 15 is a sectional view taken along the line XV-XV in FIG. 14, and shows the section perpendicular to the open and close directions of the first jaw 21. As shown in FIG. 13 to FIG. 15, in the present embodiment as well, the frame member 42 includes the plate-shaped base portion 61, and the grasping surface side end face (close direction side end face) 51 of the frame member 42 (the jaw main body 41) is formed by the base portion 61. However, in the present embodiment, the frame portion 62 is not provided in the frame member 42.

In the present embodiment as well, the column extension 65 extends in the open and close directions of the first jaw 21 in the frame member 42. One end (grasping surface side end) of the column extension 65 is continuous with the base portion 61. In the present embodiment, the lattice structure 70 is formed by the column extension 65 in the section perpendicular to the open and close directions of the first jaw 21 (directions of an arrow Y1 and an arrow Y2 in each of FIGS. 13 and 14).

In the present embodiment, the space 66 is formed in the part where the column extension 65 does not extend in a region located on the back surface 47 side with respect to the base portion 61. Therefore, the space 66 in which the frame member 42 (the base portion 61 and the column extension 65) is the defining surface is defined in the region located on the back surface 47 side (on the close direction side of the first jaw 21) with respect to the base portion 61. That is, the space 66 in which the frame member 42 is the defining surface is defined by the column extension 65 between the base portion 61 and the back surface 47 of the first jaw 21.

In the present embodiment, the space 66 defined by the column extension 65 is filled with the filling material 72. In the present embodiment, the jaw main body 41 is formed by the frame member 42 and the filling material 72. As in the fourth modification of the first embodiment, the filling material 72 is made of a heat-resistant material lower in thermal conductivity than the frame member 42. For example, the filling material 72 is made of a porous metal including at least one of steel, aluminum, stainless, and titanium, or a foamed resin including at least one of polyether ether ketone (PEEK) and polyimide (PI). In the present embodiment, the base portion 61 and the filling material 72 cooperate to form an outer circumferential surface 73 of the jaw main body 41 surrounding (covering) the column extension 65. In the present embodiment, the frame portion 62 is not provided in the frame member 42, so that the back surface 47 of the first jaw 21 is formed by the filling material 72.

In the present embodiment, the space 66 is formed in a region located on the back surface side with respect to the base portion 61 in the frame member 42, and the space 66 is filled with the filling material 72. Therefore, the frame member 42 is not continuous without space from the grasping surface side end face 51 to the back surface 47 of the first jaw 21 in the open and close directions of the first jaw 21, and the space 66 in which the frame member 42 is the defining surface is defined between the grasping surface side end face 51 and the back surface 47 of the first jaw 21. The space 66 formed by the frame member 42 is filled with the filling material 72. The filling material 72 that fills the space 66 is low in thermal conductivity. Thus, in the present embodiment as well, it is difficult for heat to be transferred to parts other than the grasping surface 46 in the outer surface of the first jaw 21 in a state where a treatment is conducted in the vicinity of the grasping surface 46.

In the present embodiment as well, the column extension 65 extends in the region located on the back surface side with respect to the base portion 61 in the frame member 42, and one end (grasping surface side end) of the column extension 65 is continuous with the base portion 61. Thus, even if the space 66 is formed in the region located on the back surface 47 side with respect to the base portion 61, the rigidity of the frame member 42 is not lower, and the rigidity of the first jaw 21 is not lower either.

Therefore, in the present embodiment as well, low performance of heat transfer to parts other than the grasping surface 46 is maintained in the outer surface of the first jaw 21, and the rigidity of the first jaw 21 is also ensured.

### (Modifications of Second Embodiment)

The honeycomb structure 71 may be formed by the column extension 65 in the section perpendicular to the open and close directions of the first jaw 21 as in the first modification of the first embodiment, or the column extension 65 may be only formed by the axial direction element 67A as in the second modification of the first embodiment.

In the second embodiment and its modifications, in the first jaw (21), the frame member (42) includes the plate-shaped base portion (61) which forms the grasping surface side end face (51) in the frame member (42), and the column extension (65) having one end continuous with the base portion (61). In the region located on the back surface (47) side with respect to the base portion (61), the space (66) in which the frame member (42) is the defining surface is formed, and the space (66) is filled with the filling material (72). The filling material (72) is made of a material lower in thermal conductivity than the frame member (42), and cooperates with the frame member (42) to form the outer circumferential surface (73) surrounding the column extension (65).

### (Modifications of First Embodiment and Second Embodiment)

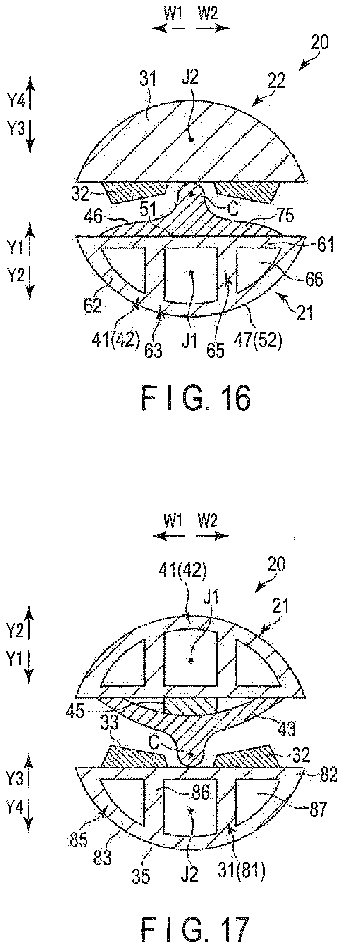

Although the first jaw 21 including the frame member 42 which defines the space 66 is rotatable relative to the shaft 5 in the above embodiments and others, it is not limited to this. For example, in a first modification of the first embodiment and the second embodiment shown in FIG. 16, the first jaw 21 may be fixed to the shaft 5, and the second jaw 22 may be rotatable relative to the shaft 5. In the present modification as well as in the first embodiment and the second embodiment, the second jaw 22 includes the jaw main body (support member) 31 and the electrode portion (second electrode portion) 32.

In the present modification, in the first jaw 21, the blade 43 and the heat generator 45 are not provided, and an electrode portion (first electrode portion) 75 is provided as an energy feeding portion instead. The grasping surface 46 of the first jaw 21 is formed by the electrode portion 75. The electrode portion 75 can abut on the jaw main body 31 of the second jaw 22, and in a state where the electrode portion 75 is in abutment with the first jaw 21, the electrode portion 75 of the first jaw 21 does not contact the electrode portion (second electrode portion) 32 of the second jaw 22. High-frequency electric power output from the energy source unit 10 is supplied to the electrode portion 75 of the first jaw 21. The treated target is treated with the high-frequency electric current, as has been described in the first embodiment, by the supply of the high-frequency electric power to the electrode portion 75 and the electrode portion 32. That is, in the present modification, the high-frequency electric power (high-frequency electric current) is fed to the living tissue as energy for use in the treatment from the grasping surface 46 by the electrode portion 75 which is the energy feeding portion.

In the present modification as well, the jaw main body 41 of the first jaw 21 includes the frame member 42. The electrode portion (first electrode portion) 75 is attached to the grasping surface side end face 51 of the frame member 42. In the present modification, as in the first embodiment, the frame member 42 includes the base portion 61, the frame portion 62, and the column extension 65. Thus, the space 66 is formed inside the outer frame 63 of the frame member 42 by the column extension 65.

In a certain modification, the electrode portion 75 may be provided in the first jaw 21, and the jaw main body (first jaw main body) 41 may be formed to have a configuration similar to that in one of the modifications of the first embodiment, the second embodiment, and the modifications of the second embodiment.

As in a second modification of the first embodiment and the second embodiment shown in FIG. 17, the frame member 42 may be provided in the jaw main body (first jaw main body) 41 of the first jaw 21, and a frame member 81 may be provided in the jaw main body (second jaw main body) 31 of the second jaw 22. In the present modification, the frame member (first frame member) 42 is formed to have a configuration similar to that in the first embodiment.

The frame member (second frame member) 81 is formed to have, for example, a configuration similar to that of the frame member 42 in the first embodiment. That is, the frame member 81 includes a base portion 82 and a frame portion 83, and an outer frame 85 is formed by the base portion 82 and the frame portion 83. A column extension 86 extends inside the outer frame 85, and one end (grasping surface side end) of the column extension 86 is continuous with the base portion 82. Inside the outer frame 85, a space 87 in which the frame member 81 is a defining surface is formed by the column extension 86. Consequently, in the present modification, low performance of heat transfer to parts other than the grasping surface 33 is maintained in the outer surface of the second jaw 22, and the rigidity of the second jaw 22 is also ensured. That is, advantageous effects similar to those of the first jaw 21 are provided in the second jaw 22 as well.

In a certain modification, the frame member 81 of the second jaw 22 may be formed to have a configuration similar to that of the frame member 42 in one of the modifications of the first embodiment, the second embodiment, and the modifications of the second embodiment.

In the embodiments described above, one of the two jaws (21 and 22) (e.g. the second jaw 22) is fixed to the shaft 5, and the other of the two jaws (21 and 22) (e.g. the first jaw 21) is rotatable relative to the shaft 5 in the above embodiments and others. However, it is not limited to this. In a certain modification, both the first jaw 21 and the second jaw 22 may be rotatably attached to the shaft 5. In this case, if the rod 25 is moved along the longitudinal axis C, both the first jaw 21 and the second jaw 22 rotate relative to the shaft 5. Consequently, the space between the first jaw 21 and the second jaw 22 opens or closes in the grasping treatment unit 20.

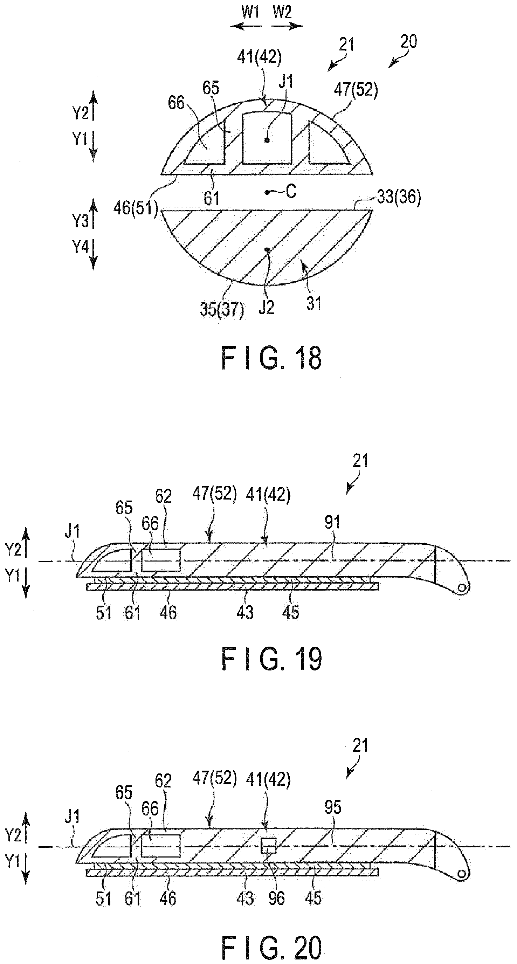

As in a third modification of the first embodiment and the second embodiment shown in FIG. 18, the first jaw 21 may be only formed by the jaw main body 41 (the frame member 42 in the present modification). In the present modification as well, the frame member 42 includes the base portion 61 and the column extension 65, and the space 66 in which the frame member 42 is the defining surface is defined.

In the present modification, the grasping surface side end face 51 of the frame member 42 serves as the grasping surface 46 of the first jaw 21. That is, the grasping surface 46 is formed by the base portion 61 of the frame member 42.

In the present modification, the second jaw 22 is only formed by the jaw main body (second jaw main body) 31. Because the jaw main body 31 is formed into a continuous wall shape (solidly) without space from the grasping surface side end face 36 to the back surface side end face 37, the second jaw 22 is continuous without space from the grasping surface 33 to the back surface 35. In the present modification, a vibration generator (not shown) such as an ultrasonic transducer is provided inside the holding unit 3. The energy source unit 10 includes a conversion circuit which converts electric power from the electric power supply into vibration generating electric power, and the vibration generating electric power (vibration generating electric energy) output from the energy source unit 10 is supplied to the vibration generator. As a result, ultrasonic vibration is generated in the vibration generator, and the ultrasonic vibration is transmitted to the second jaw 22.

When the second jaw 22 vibrates due to the ultrasonic vibration in a state where the treated target is grasped between the first jaw 21 and the second jaw 22, the treated target is coagulated and cut open at the same time. In this instance, heat is generated in the vicinity of the grasping surface 46 of the first jaw 21. In the present modification as well, the frame member 42 having a configuration similar to that in the first embodiment is provided in the first jaw 21, and it is therefore difficult for heat to be transferred to parts other than the grasping surface 46 in the outer surface of the first jaw 21. Because the frame member 42 having a configuration similar to that in the first embodiment is provided in the first jaw 21, the rigidity of the first jaw 21 is also ensured.

In a certain modification, the first jaw 21 may be only formed by the jaw main body 41, and the jaw main body (first jaw main body) 41 may be formed to have a configuration similar to that in one of the modifications of the first embodiment, the second embodiment, and the modifications of the second embodiment.

The configuration of the frame member 42 in the above embodiments and others is applicable to the grasping treatment unit 20 which treats the treated target grasped between the first jaw 21 and the second jaw 22 by use of heat, a high-frequency, ultrasonic vibration, or the like. Therefore, in the energy feeding portion (43; 75) provided in the first jaw 21, at least one of heat, a high-frequency, and ultrasonic vibration has only to be fed to the treated target as energy for use in the treatment via the grasping surface 46. In a certain embodiment, energy (e.g. microwaves or laser) different from heat, a high-frequency, and ultrasonic vibration has only to be fed to the treated target.

In the first embodiment, the second embodiment, and their modifications, the grasping treatment unit (20) includes the first jaw (21) extending from the proximal portion to the distal portion, and the second jaw (22) which extends from the proximal portion to the distal portion and which is openable and closable relative to the first jaw (21). The outer surface of the first jaw (21) is provided with the grasping surface (46) facing the second jaw (22), and the back surface (47) facing the side opposite to the grasping surface (46). The frame member (42) is provided in the first jaw (21), and the frame member (42) includes the plate-shaped base portion (61) which forms the grasping surface side end face (51) of the frame member (42). In the frame member (42), the column extension (65) extends from the base portion (61) toward the back surface (47), and one end of the column extension (65) is continuous with the base portion (61). Between the base portion (61) and the back surface (47) of the first jaw (21), the space (66) in which the frame member (42) is the defining surface is defined by the column extension (65).

### (Third Embodiment)

Next, a third embodiment of the present invention will be described with reference to FIG. 19. The third embodiment shows the following modification of the configuration of the first embodiment. The same parts as those in the first embodiment are provided with the same reference marks, and are not described.

FIG. 19 is a diagram showing the first jaw 21 in the section perpendicular to the width directions. In the present embodiment as well in the first embodiment, the jaw main body (first jaw main body) 41 of the first jaw 21 includes the frame member 42. The base portion 61 and the column extension 65 are provided in the frame member 42, and the space 66 in which the frame member 42 is the defining surface is defined by the column extension 65 between the base portion 61 and the back surface 47 of the first jaw 21. In the present embodiment as well in the first embodiment, the frame portion 62 is provided. The column extension 65 extends inside the base portion 61 and the frame portion 62, and the space 66 is formed inside the base portion 61 and the frame portion 62. The lattice structure 70 is formed by the column extension 65 in the section perpendicular to the open and close directions of the first jaw 21.

However, in the present embodiment, the base portion 61, the frame portion 62, and the column extension 65 are only provided in the distal-side part of the jaw main body 41 (the frame member 42). For example, the base portion 61, the frame portion 62, and the column extension 65 are only provided in the part on the distal side with respect to the intermediate position in the longitudinal direction of the first jaw 21 (first jaw longitudinal direction). Therefore, the space 66 defined by the column extension 65 is only formed in the distal-side part of the frame member 42 (the jaw main body 41). In the distal-side part of the frame member 42, the grasping surface side end face 51 of the frame member 42 is formed by the base portion 61, and the back surface side end face 52 of the frame member 42 (the back surface 47 of the first jaw 21 in the present embodiment) is formed by the frame portion 62.

In the present embodiment, a wall portion 91 is provided in the proximal-side part of the frame member 42 (the jaw main body 41). For example, the wall portion 91 is only provided in the part on the proximal side with respect to the intermediate position in the longitudinal direction of the first jaw 21 (first jaw longitudinal direction). Therefore, the wall portion 91 is located on the proximal side with respect to the base portion 61, the frame portion 62, and the column extension 65 in the frame member 42. In the wall portion 91, the frame member 42 is continuous without space from the grasping surface side end face 51 to the back surface side end face 52. Therefore, in the proximal-side part of the frame member 42, the grasping surface side end face 51 and the back surface side end face 52 are formed by the wall portion 91.

Since the frame member 42 is formed as described above, the space is only formed in the distal-side part in the frame member 42 according to the present embodiment. Thus, the jaw main body 41 is lower in thermal conductivity in the distal-side part than in the proximal-side part. The jaw main body 41 is higher in rigidity in the proximal-side part than in the distal-side part.

When a treatment is conducted by use of energy such as heat or a high-frequency, there is a strong possibility that the distal-side part may contact a living tissue or the like other than the treated target in the back surface 47 of the first jaw 21. In the present embodiment, thermal conductivity is lower in the distal-side part of the frame member 42. It is thus difficult for heat to be transferred to the outer surface other than the grasping surface 46 in the distal-side part of the first jaw 21. Low performance of heat transfer to the outer surface other than the grasping surface 46 is maintained in the distal-side part of the first jaw 21, which ensures that the influence of heat on the living tissue other than the treated target can be reduced.

When the treated target is grasped between the first jaw 21 and the second jaw 22, the proximal-side part is heavily loaded in the first jaw 21. In the present embodiment, rigidity is higher in the proximal-side part of the frame member 42, so that rigidity is also higher in the proximal-side part of the first jaw 21. As a result, the deformation of the first jaw 21 is more difficult, and a great grasping force amount is maintained in a state where the treated target is grasped between the first jaw 21 and the second jaw 22. Therefore, treatment performance can be improved when the treated target grasped between the first jaw 21 and the second jaw 22 is treated.

### (Modifications of Third Embodiment)

Even in the configuration in which the wall portion 91 is provided in the proximal-side part of the frame member 42, the honeycomb structure 71 may be formed by the column extension 65 in the section perpendicular to the open and close directions of the first jaw 21 as in the first modification of the first embodiment, or the column extension 65 may be only formed by the axial direction element 67A as in the second modification of the first embodiment. As in the third modification of the first embodiment, the other end (back surface side end) of the column extension 65 may be non-continuous with the frame portion 62. As in the fourth modification of the first embodiment, the space 66 may be filled with the filling material 72. Even in the configuration in which the wall portion 91 is provided in the proximal-side part of the frame member 42, no frame portion 62 may be provided in the frame member 42, and the space 66 may be filled with the filling material 72 in the region located on the back surface side with respect to the base portion 61, as in the second embodiment and its modifications. In the configuration in which the grasping treatment unit 20 is formed as shown in each of the modifications of the first embodiment and the second embodiment, the first jaw 21 provided with the wall portion 91 in the proximal-side part of the frame member 42 may be used as in the third embodiment. The energy to be fed to the treated target may be at least one of heat, a high-frequency, and ultrasonic vibration, or energy (e.g. microwaves or laser) different from heat, a high-frequency, and ultrasonic vibration may be fed to the treated target.

In the third embodiment and its modifications, the grasping treatment unit (20) includes the first jaw (21) extending from the proximal portion to the distal portion, and the second jaw (22) which extends from the proximal portion to the distal portion and which is openable and closable relative to the first jaw (21). The outer surface of the first jaw (21) is provided with the grasping surface (46) facing the second jaw (22), and the back surface (47) facing the side opposite to the grasping surface (46). The jaw main body (41) including the frame member (42) is provided in the first jaw (21), and the frame member (42) includes the plate-shaped base portion (61) which forms the grasping surface side end face (51) in the distal-side part of the frame member (42). In the distal-side part of the frame member (42), the column extension (65) extends from the base portion (61) toward the back surface (47), and one end of the column extension (65) is continuous with the base portion (61). In the distal-side part of the jaw main body (41), the space (66) in which the frame member (42) is the defining surface is defined by the column extension (65) between the base portion (61) and the back surface (47) of the first jaw (21). In the frame member (42), the wall portion (91) is provided on the proximal side with respect to the base portion (61) and the column extension (65). In the wall portion (91), the frame member (42) is continuous without space from the grasping surface side end face (51) to the back surface side end face (52).

Owing to the configuration described above, in the third embodiment and its modifications, thermal conductivity is lower in the distal-side part than in the proximal-side part of the jaw main body (41) of the first jaw (21), and rigidity is higher in the proximal-side part than in the distal-side part.

### (Fourth Embodiment)

Next, a fourth embodiment of the present invention will be described with reference to FIG. 20. The fourth embodiment shows the following modification of the configuration of the third embodiment. The same parts as those in the third embodiment are provided with the same reference marks, and are not described.

In the third embodiment, in the wall portion 91 provided in the frame member 42, the frame member 42 is continuous without space from the grasping surface side end face 51 to the back surface side end face 52. In contrast, according to the present embodiment, a wall portion 95 is provided instead of the wall portion 91 in a region located on the proximal side with respect to the base portion 61 and the column extension 65 in the frame member 42. In the present embodiment, one cavity 96 is formed in the wall portion 95 between the grasping surface side end face 51 and the back surface side end face 52 of the frame member 42. The cavity 96 is surrounded by the wall portion 95. Therefore, in the configuration according to the present embodiment, the frame member 42 is not continuous without space between the grasping surface side end face 51 and the back surface side end face 52 in the wall portion 95.

However, in the present embodiment as well, the wall portion 95 is provided on the proximal side with respect to the base portion 61 and the column extension 65 in the frame member 42 (the jaw main body) 41. In the frame member 42 (the jaw main body) 41, the wall portion 95 is higher in rigidity than the distal-side part of the frame member 42 in which the base portion 61 and the column extension 65 are provided. That is, the volume of the cavity 96 formed in the wall portion 95 is low. Thus, in the present embodiment as well as in the third embodiment, rigidity is higher in the proximal-side part in the first jaw 21 as well. Consequently, in the present embodiment as well, the deformation of the first jaw 21 is more difficult, and a great grasping force amount is certainly maintained in a state where the treated target is grasped between the first jaw 21 and the second jaw 22.

### (Modifications of Fourth Embodiment)

Although only one cavity 96 is formed in the wall portion 95 in the fourth embodiment, more than one cavity (96) may be formed between the grasping surface side end face 51 and the back surface side end face 52 in the wall portion 95 in a certain modification. However, in this case as well, the total of the volumes of the cavities (96) is small, the wall portion 95 is higher in rigidity than the distal-side part of the frame member 42 in which the base portion 61 and the column extension 65 are provided. Therefore, as in the fourth embodiment, rigidity is higher in the proximal-side part in the first jaw 21.

### (Fifth Embodiment)

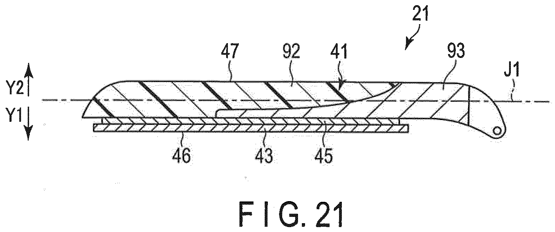

Next, a fifth embodiment of the present invention will be described with reference to FIG. 21. The fifth embodiment shows the following modification of the configuration of the third embodiment. The same parts as those in the third embodiment are provided with the same reference marks, and are not described.

FIG. 21 is a diagram showing the first jaw 21 in the section perpendicular to the width directions. As shown in FIG. 21, the frame member 42 is not provided in the jaw main body 41 in the present embodiment. Instead, in the present embodiment, the jaw main body 41 is formed by a first material portion 92 and a second material portion 93. The first material portion 92 is made of a first material such as a resin including at least one of polyether ether ketone and polyimide. The second material portion 93 is made of a second material higher in thermal conductivity than the first material and higher in rigidity than the first material. For example, the second material portion 93 is made of a metal including at least one of stainless and titanium. In the present embodiment, the back surface 47 of the first jaw 21 is formed by the jaw main body 41.

In the jaw main body 41, the proportion of the first material portion 92 to the second material portion 93 is higher in the distal-side part than in the proximal-side part. Thus, the content of the first material is higher in the distal-side part of the jaw main body 41 than in the proximal-side part of the jaw main body 41. Therefore, in the jaw main body 41 of the first jaw 21, thermal conductivity is lower in the distal-side part than in the proximal-side part, and rigidity is higher in the proximal-side part than in the distal-side part.

In the present embodiment as well as in the third embodiment, thermal conductivity is lower in the distal-side part of the jaw main body (first jaw main body) 41. It is thus difficult for heat to be transferred to the outer surface other than the grasping surface 46 in the distal-side part of the first jaw 21. In the present embodiment as well as in the third embodiment, rigidity is higher in the proximal-side part of the jaw main body 41, so that rigidity is higher in the proximal-side part in the first jaw 21 as well. Consequently, the deformation of the first jaw 21 is more difficult, and a great grasping force amount is certainly maintained in a state where the treated target is grasped between the first jaw 21 and the second jaw 22.

### (Modifications of Fifth Embodiment)

In the configuration in which the grasping treatment unit 20 is formed as shown in each of the modifications of the first embodiment and the second embodiment, the first jaw 21 in which the jaw main body 41 is formed by the first material portion 92 and the second material portion 93 may be used as in the fifth embodiment. The energy to be fed to the treated target may be at least one of heat, a high-frequency, and ultrasonic vibration, or energy (e.g. microwaves or laser) different from heat, a high-frequency, and ultrasonic vibration may be fed to the treated target.

In the fifth embodiment and its modifications, the jaw main body (41) is made of the first material, and the second material higher in thermal conductivity and higher in rigidity than the first material. The content of the first material is higher in the distal-side part of the jaw main body (41) than in the proximal-side part of the jaw main body (41). Thus, in the fourth embodiment and its modifications, in the jaw main body (41) of the first jaw (21), thermal conductivity is lower in the distal-side part than in the proximal-side part, and rigidity is higher in the proximal-side part than in the distal-side part.

Although the embodiments, etc. of the present invention have been described above, the present invention is not limited to the above-described embodiments, etc., and, needless to say, various modifications can be made without departing from the spirit of the invention.

Characteristic matters are additionally set forth below.

### Notes

### (Additional note 1)

A grasping treatment unit comprising:
a first jaw extending from a proximal portion toward a distal portion;
a second jaw which extends from a proximal portion toward a distal portion, and which is openable and closable relative to the first jaw;
a grasping surface facing the second jaw in an outer surface of the first jaw;
a back surface facing a side opposite to the grasping surface in the outer surface of the first jaw; and
a jaw main body which is provided in the first jaw, and which is lower in thermal conductivity in a distal-side part than in a proximal-side part and higher in rigidity in the proximal-side part than in the distal-side part.

### (Additional note 2)

The grasping treatment unit according to additional note 1, wherein
the jaw main body includes a frame member, and
the frame member includes
a plate-shaped base portion which forms a grasping surface side end face of the frame member in the distal-side part of the jaw main body,
a column extension which extends from the base portion toward the back surface in the distal-side part of the jaw main body, and which has one end continuous with the base portion, the column extension defining a space in which the frame member is a defining surface between the base portion and the back surface of the first jaw in the distal-side part of the jaw main body, and
a wall portion which is provided on a proximal side with respect to the base portion and the column extension, and in which the frame member is continuous without space from the grasping surface side end face to a back surface side end face of the frame member.

### (Additional note 3)

The grasping treatment unit according to additional note 2, wherein the jaw main body includes a frame portion which forms the back surface side end face of the frame member in the distal-side part of the jaw main body, and which cooperates with the base portion to form an outer frame of the jaw main body, the column extension extending inside the outer frame.

### (Additional note 4)

The grasping treatment unit according to additional note 1, wherein the jaw main body is made of a first material, and a second material higher in thermal conductivity and higher in rigidity than the first material, and
the content of the first material is higher in the distal-side part of the jaw main body than in the proximal-side part of the jaw main body.

### (Additional note 5)

The grasping treatment unit according to additional note 4, wherein the first material is a resin including at least one of polyether ether ketone and polyimide, and
the second material is a metal including at least one of stainless and titanium.

## Claims

1. A grasping treatment unit comprising:
a first jaw extending from a proximal portion toward a distal portion;
a second jaw which extends from a proximal portion toward a distal portion, and which is openable and closable relative to the first jaw;
a grasping surface facing the second jaw in an outer surface of the first jaw;
a back surface facing a side opposite to the grasping surface in the outer surface of the first jaw;
a frame member provided in the first jaw;
a plate-shaped base portion which forms a grasping surface side end face in the frame member; and
a column extension which extends from the base portion toward the back surface in the frame member, and which has one end continuous with the base portion, the column extension defining a space in which the frame member is a defining surface between the base portion and the back surface of the first jaw.

2. The grasping treatment unit according to claim 1, wherein the frame member includes a frame portion which has both ends continuous with the base portion, and which cooperates with the base portion to form an outer frame of the frame member, the space being defined inside the outer frame and the column extension extending inside the outer frame.

3. The grasping treatment unit according to claim 2, wherein the other end of the column extension is continuous with the frame portion.

4. The grasping treatment unit according to claim 2, wherein the first jaw includes a filling material which fills the space defined by the column extension inside the outer frame of the frame member, and which is made of a material lower in thermal conductivity than the frame member.

5. The grasping treatment unit according to claim 4, wherein the filling material is made of a porous metal including at least one of steel, aluminum, stainless, and titanium, or a foamed resin including at least one of polyether ether ketone and polyimide.

6. The grasping treatment unit according to claim 2, wherein the column extension forms at least one of a lattice structure and a honeycomb structure in a section perpendicular to open and close directions of the first jaw.

7. The grasping treatment unit according to claim 2, wherein the frame portion forms the back surface of the first jaw.

8. The grasping treatment unit according to claim 2, wherein the frame portion is made of a metal including at least one of stainless and titanium, or a heat-resistant resin including at least one of polyether ether ketone and polyimide.

9. The grasping treatment unit according to claim 1, wherein the first jaw includes a filling material which fills the space defined by the column extension in a region on a back surface side with respect to the base portion, and which cooperates with the base portion to form an outer circumferential surface surrounding the column extension, the filling material being made of a material lower in thermal conductivity than the frame member.

10. The grasping treatment unit according to claim 9, wherein the filling material is made of a porous metal including at least one of steel, aluminum, stainless, and titanium, or a foamed resin including at least one of polyether ether ketone and polyimide.

11. The grasping treatment unit according to claim 9, wherein the column extension forms at least one of a lattice structure and a honeycomb structure in a section perpendicular to open and close directions of the first jaw.

12. The grasping treatment unit according to claim 9, wherein the filling material forms the back surface of the first jaw.

13. The grasping treatment unit according to claim 1, wherein a hole which penetrates the base portion in open and close directions of the first jaw is formed in the base portion.

14. The grasping treatment unit according to claim 1, wherein the frame member is made of a metal including at least one of stainless and titanium, or a heat-resistant resin including at least one of polyether ether ketone and polyimide.

15. The grasping treatment unit according to claim 1, wherein the first jaw includes an energy feeding portion which forms the grasping surface, and which is coupled to a grasping surface side of the frame member, the energy feeding portion being configured to feed energy for use in a treatment from the grasping surface.

16. The grasping treatment unit according to claim 15, wherein the energy feeding portion is configured to feed at least one of heat, a high-frequency, and ultrasonic vibration via the grasping surface as the energy for use in the treatment.

17. The grasping treatment unit according to claim 1, wherein the base portion forms the grasping surface of the first jaw.

18. The grasping treatment unit according to claim 1, wherein the base portion and the column extension are provided in a distal-side part of the first jaw, and
the frame member includes a wall portion which is provided on a proximal side with respect to the base portion and the column extension, and in which the frame member is continuous without space from the grasping surface side end face to a back surface side end face of the frame member.

19. The grasping treatment unit according to claim 1, wherein the base portion and the column extension are provided in a distal-side part of the first jaw,
the frame member includes a wall portion which is provided on the proximal side with respect to the base portion and the column extension, and which is higher in rigidity than the distal-side part where the base portion and the column extension are provided, and
the wall portion has at least one cavity surrounded by the wall portion between the grasping surface side end face and a back surface side end face of the frame member.

20. A grasping treatment instrument comprising:
the grasping treatment unit according to claim 1;
a holding unit which is provided on a proximal side with respect to the grasping treatment unit, and which is holdable; and
an open-or-close operation input portion which is provided in the holding unit, and to which an open-or-close operation to open or close a space between the first jaw and the second jaw of the grasping treatment unit is input.
